# EUROPEAN PATENT SPECIFICATION

(11) **EP 3 806 809 B1**
(45) Date of publication and mention of the grant of the patent: **28.01.2026**
(21) Application number: 19735060.6
(22) Date of filing: 17.06.2019
(51) Int. Cl.: A61J 1/20

(54) **RECONSTITUTION SYSTEM TO ADMINISTER A DRUG VIA A HIGH VACUUM VIAL**
REKONSTITUTIONSSYSTEM ZUR VERABREICHUNG EINES ARZNEIMITTELS ÜBER EIN HOCHVAKUUM-VIAL
SYSTÈME DE RECONSTITUTION POUR ADMINISTRER UN MÉDICAMENT VIA UN FLACON À VIDE POUSSÉ

(30) Priority: 18.06.2018 US 201816011076
(43) Date of publication of application: 21.04.2021
(73) Proprietor: Baxter International Inc., Deerfield, Illinois 60015-4633 (US); BAXTER HEALTHCARE SA, 8152 Glattpark (Opfikon) (CH)
(72) Inventor: DESBROSSES, Freddy, 6530 Thuin (BE); PADULA, Pierpaolo, 1040 Etterbeek (BE); ABDESSALEM, Yannis, 1050 Ixelles (BE); DESRIAUX, Marjorie, 1040 Etterbeek (BE); PORQUERAS, Diego Santo-Domingo, 1050 Ixelles (BE)
(74) Representative: Potter Clarkson
(86) International application number: PCT/US2019/037468
(87) International publication number: WO 2019/245960

(56) References cited:
- EP-A1- 2 842 540
- WO-A1-2019/032934
- WO-A2-2011/071952

## Description

### BACKGROUND

Medical therapy often requires the intravenous administration of, for example, fluids to correct electrolyte imbalance, medicated solutions to deliver medication, and/or nutrients to provide nutrition to patients unable to receive oral or enteral nutrition. Intravenous ("IV") fluids are generally available in aseptic single- or multi-chamber flexible containers that include an administration port and a medication port. An IV administration set is typically inserted into a flexible container (e.g., an IV bag) via the administration port to allow administration of the fluids from the flexible container to the patient. Likewise, medications and/or nutrients can be injected, into the flexible container, via the medication port.

Certain active agents, such as medications and nutrients, which may be unstable in liquid form, are stored in dry form. For example, active agents may be unstable at the pH of the IV fluid, susceptible to light, or have other instability, thus requiring dry form storage. Typically, dry form active agents may be stored in glass vials, sealed with rubber stoppers, or in other containers such as plastic containers, ampoules, or in small bags that may be closed with standard screw caps. Prior to being administered to a patient, these dry form active agents are reconstituted. Reconstitution typically includes removing a protective cover to expose the rubber stopper, wiping the stopper with an antiseptic wipe, adding a diluent to the vial by inserting a needle of a syringe through the rubber stopper and depositing the contents of the syringe, such as the diluent, into the vial, and shaking the vial to fully dissolve or suspend the active agents. Subsequently, the resulting reconstituted solution or suspension is withdrawn from the vial by inserting a needle of a syringe through the rubber stopper, aspirating the solution or suspension into the syringe, and injecting the contents of the syringe into the flexible container via the medication port. Some medications and nutrients, however, begin to degrade as soon as they are mixed into the flexible container. Ascorbic acid (Vitamin C) and Vitamin B₁, for example, may degrade in total parenteral nutrition mixtures, while Vitamins A and E may degrade in parenteral nutrition mixtures upon exposure to light.

WO 2019/032934, which was published after the priority date of the present application, discloses a reconstitution device having a piercing member (30) with first (32) and second (34) pointed ends that are configured to engage respectively an administration port of an IV bag and a vial. A fluid pathway (36) is also provided between the two ends to allow fluid to pass from the IV bag to the vial to reconstitute a powdered drug in the vial. The device also comprises a delivery member (40) having first (42) and second (44) ends that are configured to engage respectively a delivery site and the vial and a fluid pathway 46 between the two ends to allow the reconstituted drug to flow from the vial through the delivery member to the delivery site. The vial is connected to the reconstitution device top uppermost and fluid from the IV bag flows into the vial by gravity.

An improved system and method for using a reconstitution device to administer medications, especially quickly degrading medications, is needed accordingly.

### SUMMARY

To improve the administration of medications, especially those that degrade quickly, a new patient medication delivery paradigm is provided herein. To implement a new and alternate way of medication administration, a new and alternate reconstitution device, system, and method is disclosed. More specifically, a reconstitution device is provided to establish fluid pathways between a source (e.g., an intravenous ("IV") bag) and a location (e.g., a patient). Along the fluid pathways, a drug (e.g., a powdered drug, a lyophilized drug, or a liquid drug) is introduced via reconstitution device connectivity with a drug container, such as a drug vial.

According to a first aspect of the present invention, there is provided a reconstitution device according to claim 1.

In a first embodiment of the reconstitution device of the first aspect of the present invention, the cap is formed integrally with the collar.

In a second embodiment of the reconstitution device of the first aspect of the present invention, which may also be combined with the first embodiment, the collar is cylindrical and is configured to concentrically engage the vial.

In a third embodiment of the reconstitution device of the first aspect of the present invention, which may also be combined with the first or second embodiment, the administration port is an intravenous ("IV") bag port and the first end of the first piercing member is configured to pierce the IV bag port to place first piercing member in fluid communication with the IV bag port.

In a fourth embodiment of the reconstitution device of the first aspect of the present invention, which may also be combined with any of the first to third embodiments, the reconstitution device further includes an IV line.

In a fifth embodiment of the reconstitution device of the first aspect of the present invention, which may also be combined with any of the first to fourth embodiments, when placed in an operating position, each of the first piercing member and the second piercing member are oriented vertically.

In a sixth embodiment of the reconstitution device of the first aspect of the present invention, which may also be combined with any of the first to fifth embodiments the first end of the first piercing member is formed integrally with the administration port.

In a seventh embodiment of the reconstitution device of the first aspect of the present invention, which may also be combined with any of the first to sixth embodiments, the administration port is connected to an IV solution container.

In an eighth embodiment of the reconstitution device of the first aspect of the present invention, which may also be combined with any of the first to seventh embodiments, at least one of the first end of the first piercing member, the second end of the first piercing member, and the first end of the second piercing member includes or defines a syringe needle or a plastic spike.

In a ninth embodiment of the reconstitution device of the first aspect of the present invention, which may also be combined with any of the first to eighth embodiments, the vial is initially sealed and under a vacuum.

According to a second aspect of the present invention, there is provided a reconstitution system according to claim 8.

In a first embodiment of the reconstitution system of the second aspect of the present invention, the drug vial contains one of a pharmaceutical agent or a nutritional supplement.

In a second embodiment of the reconstitution system of the second aspect of the present invention, which may also be combined with the first embodiment, the drug vial is initially sealed and under a vacuum.

In a third embodiment of the reconstitution system of the second aspect of the present invention, which may also be combined with the first or second embodiment, the reconstitution system further includes an infusion pump in operable communication with the IV line.

In a fourth embodiment of the reconstitution system of the second aspect of the present invention, which may also be combined with any of the first to third embodiments, the drug vial is formed of an ultraviolet ("UV") light blocking material.

According to a third aspect of the present invention, there is provided a drug reconstitution method according to claim 13

In light of the disclosure and aspects set forth herein, it is accordingly an advantage of the present disclosure to provide a reconstitution system that ensures complete infusion of a drug from a drug vial via gravity by implementing an upside-down vial.

It is another advantage of the present disclosure to provide a reconstitution system that reduces drug degradation by reconstituting the drug and immediately delivering it to the IV administration set. The particular system may be further configured to limit environmental degradation by avoiding solution-interaction with outside factors, such as light or air. Immediate delivery may further ensure that the reconstituted drug is delivered during the first part of therapy, thus avoiding incomplete delivery that may be associated with premature therapy interruption.

It is a further advantage of the present disclosure to provide a reconstitution system that ensures the contents of the vial, such as the reconstituted drug, are diluted prior to being administrated to the patient to prevent any medical or efficacy risks based on osmolarity.

It is yet another advantage of the present disclosure to provide a ready-to-use reconstitution system configured to accept vials directly.

It is yet a further advantage of the present disclosure to provide a reconstitution system avoiding tedious preparation steps involved with typical dried drug reconstitution by accepting vials directly.

It is still a further advantage of the present disclosure to pre-plug the vial to ensure that drug prescriptions and deliveries are not inadvertently missed by medical professionals.

It is still another advantage of the present disclosure to provide a reconstitution system ensuring passive reconstitution by connecting the drug vial with a delivery site, such that, in one embodiment, no additional action is required for reconstitution and administration to take place.

In yet another advantage of the present disclosure, each discrete component of the reconstitution system may be processed, sterilized, and handled separately on a componentby-component basis.

Moreover, an advantage of the present disclosure is to provide a reconstitution system ensuring that microbiological contamination risk is minimized by accepting pre-plugged vials in a sterile manner.

Additional features and advantages of the disclosed devices, systems, and methods are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not necessarily have to have all of the advantages listed herein. Moreover, it should be noted that the language used in the specification has been selected for readability and instructional purposes, and not to limit the scope of the inventive subject matter.

### BRIEF DESCRIPTION OF THE FIGURES

Understanding that figures depict only typical embodiments of the invention and are not to be considered to be limiting the scope of the present disclosure, the present disclosure is described and explained with additional specificity and detail through the use of the accompanying figures. The figures are listed below.
Fig. 1 is an exploded-sectioned view of one embodiment of a reconstitution device, according to an example embodiment of the present disclosure.
Fig. 2 is a sectioned view of the reconstitution device of Fig. 1, further illustrating device engagement, according to an example embodiment of the present disclosure.
Fig. 3 is an elevation-sectioned view of the reconstitution device of Fig. 1, further illustrating device engagement, according to an example embodiment of the present disclosure.
Fig. 4 is an elevation-sectioned view of one alternative embodiment of a reconstitution device of the present disclosure, which includes an integrated fluid container.
Fig. 5 is a schematic view of one embodiment of a system employing any of the reconstitution devices described herein.
Fig. 6 is a plot of osmolarity profile as a function of time, as performed by a reconstitution device of the present disclosure.
Fig. 7 is a plot of reconstituted drug infusion as a function of time, as performed by a reconstitution device of the present disclosure.

### DETAILED DESCRIPTION

Certain embodiments described herein relate generally to the field of intravenous ("IV") administration of an active agent. More particularly, some embodiments described herein relate to the reconstitution of an active agent in a vial, followed by IV administration of the active agent using an IV administration set.

As discussed herein, adding a dried drug to an IV bag typically includes several tedious steps. For example, a medical professional may be required to remove a protective cover from a vial, wipe a stopper of the vial, add diluent to the vial, and shake the vial. The drug then has to be withdrawn from the vial and injected into, for example, an IV line or an IV bag. Additionally, as discussed above, once a drug is reconstituted, the drug may begin to degrade; thus, time of infusion is important to keep at a minimum.

Typical commercially available vial reconstitution systems (e.g., the Baxter Vial-Mate^{™} system) are configured such that the drug contained in the vial is added into the IV bag directly, via a port. In such systems, the drug from the vial is mixed with the entire IV bag prior to being administered to the patient. For example, the drug is delivered into the IV bag, via a medication port, mixes within the IV bag, and is then administered to the patient via an administration port (e.g., by an IV set connected to the IV bag at the administration port). In this delivery paradigm, the drug is not fully delivered until the entire contents of the IV bag have been delivered. The longer the drug sits in the IV bag in a reconstituted state, while waiting to be delivered, the more likely it is that the drug will degrade. Furthermore, the approach of typical reconstitution devices is difficult when the IV bag is a multi-chamber bag, especially one that has been activated. In those situations, because the bag has under-fill characteristics and is often a large-format bag, it is difficult to deliver a drug into a multi-chamber bag, ensure that the drug reconstitutes in the multi-chamber bag, and subsequently deliver the reconstituted drug from the multi-chamber bag to the patient.

By comparison, the reconstitution devices, systems, and methods disclosed herein are configured so that the vial containing the drug is connected directly with the delivery site. The connection allows the reconstituted drug to travel directly to the delivery site, such as an IV set, thus avoiding the requirement of having to mix the drug in the IV bag. For example, the reconstituted drug may flow directly into the IV administration set, for direct delivery to the patient during IV therapy. By having the drug be reconstituted in and delivered from a smaller volume container, such as the drug vial itself, the time for delivery may be reduced. It is believed that such structure will reduce the likelihood that the drug will degrade prior to delivery.

The reconstitution system disclosed herein may implement several flow paths, including flow paths that are in fluid communication with the drug vial and flow paths that are not in fluid communication with the drug vial. This structural configuration may enhance the efficacy by which a drug is reconstituted in the vial and delivered subsequently to a patient. Furthermore, the vial may initially be sealed and under a vacuum. When initially reconstituted, the vacuum may further enhance the efficacy by which a drug is reconstituted in a vial and subsequently delivered to a patient.

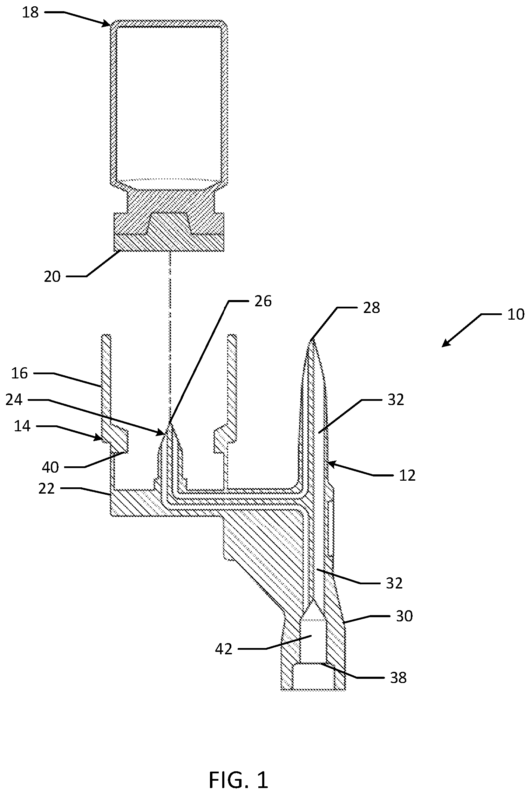

Referring now to the drawings and in particular to Fig. 1, one embodiment of the reconstitution device of the present disclosure is illustrated by reconstitution device 10. Reconstitution device 10 may be constructed of any suitable plastic or rubber material, such as polyvinyl chloride ("PVC"), non-DEHP PVC, Krayton polypropylene mixture, or other similar materials.

Reconstitution device 10 includes a first piercing member 12 and a receptacle 14. The receptacle 14 includes a cylindrical collar 16. In alternative embodiments, collar 16 may have other cross sections (e.g., hexagonal, square, triangular, or other suitable geometric shape). Cylindrical collar 16 is configured to engage a vial 18 (e.g., via concentric engagement). The vial 18 may be fitted with a stopper 20, which may be a rubber stopper, a plastic stopper, and/or include a foil cover, or be any other similar structure for sealing vial 18. Stopper 20 may protect the contents of vial 18 from environmental factors, such as ambient air. In an embodiment, vial 18 is formed of an ultraviolet ("UV") light blocking material, which may further protect the contents of vial 18 from environmental factors, such as light.

In the illustrated embodiment, the stopper-side of vial 18 may be received within cylindrical collar 16 of device 10, such that the cylindrical collar 16 is disposed around the outside of the stopper-side of vial 18. Vial 18 typically contains an active agent such as a pharmaceutical agent or a nutritional supplement. The active agent may be present as a dried powder, such as a powder obtained by lyophilization. Alternatively, the active agent is present in an aqueous solution or suspension, or other typical liquid form. As discussed herein, reconstitution device 10 is particularly suited for connecting to an IV line to deliver a reconstituted solution to a patient.

The receptacle 14 further includes a cap 22. Cap 22 may be formed integrally with or be attached to collar 16. The cap 22 extends from the collar 16 and holds a second piercing member 24. A first pointed end 26 of the second piercing member 24 is disposed within the collar 16.

The first piercing member 12 has a first pointed end 28 and a second end 30. The first piercing member 12 defines a first fluid pathway 32 that extends from the first end 28 of the first piercing member 12 to the second end 30 of the first piercing member 12. The first end 28 of the first piercing member 12 is configured to engage with an administration port of an IV bag, so that the second end 30 of the first piercing member 12 is in fluid communication with the IV bag via the first fluid pathway 32. In an embodiment, at least one of the first end 28 of the first piercing member 12, the second end 30 of the first piercing member 12, and the first end 26 of the second piercing member 24 may include or form a syringe needle or a plastic spike.

The second end 30 of the first piercing member 12 is configured to be placed into fluid communication with an IV line. In an embodiment, the second end 30 of the first piercing member 12 may include a connector (e.g., a male or female luer connector) to fluidly sealingly engage a delivery site, such as a mating luer connector of an IV line, which is in turn connected to a patient. Other types of connections, besides luer connections, such as hose barbs or compression fittings, may be used to connect the second end 30 of the first piercing member 12 to the delivery site (e.g., an IV line tube). The second end 30 of the first piercing member 12 further includes a removable membrane or a pierceable membrane 38. Though membrane 38 is described with reference to the first piercing member 12, it should be appreciated that membrane 38 is in common with both the first fluid pathway 32 and a third fluid pathway 36 (described in greater detail below). Thus, membrane 38 may prevent flow from each of the first piercing member 12 and the second piercing member 24 from pre-maturely exiting the reconstitution device 10 to the delivery site.

For example, membrane 38 is configured to temporarily block fluid flow from going past the second end 30 of the first piercing member 12. In this way, membrane 38 defines a common mixing chamber 42, whereby fluid from the first fluid pathway 32 (e.g., saline or glucose) mixes with fluid from the third fluid pathway 36 (e.g., a reconstituted drug) prior to administration to a delivery location, such as the IV line. Once a drug has been reconstituted/mixed and is ready for delivery, membrane 38 may be removed or eliminated (e.g., pierced), so that the mixing chamber 42 communicates with the IV line. Membrane 38 may further ensure a vacuum exists within the various fluid pathways communicating with vial 18 during reconstitution, to advantageously generate pressurized fluid streams for reconstitution, as described in greater detail below. Furthermore, membrane 38 may reduce or eliminate leakage from reconstitution device 10, such as when an IV solution container is attached or detached from first end 28 or when an IV line is attached or detached from second end 30.

The first end 28 of first piercing member 12 is, in the illustrated embodiment, configured to pierce an administration port (e.g., an IV bag port), so that the first end 28 of the first piercing member 12 is in fluid communication with the administration port. The administration port may be connected to an IV solution container or may be formed integrally with the IV solution container.

Reconstitution device 10 may be configured to engage with the administration port of any suitable IV bag. First piercing member 12 and second piercing member 24 may be made of metal (e.g., stainless steel), medical grade plastic, or any other suitable material. In an alternative embodiment, the first end 28 of the first piercing member 12 is formed integrally with the administration port of the IV bag. For example, in an embodiment, the IV bag may be permanently fixed to the reconstitution device 10, such that the reconstitution device 10 is manufactured and configured with the IV bag.

Reconstitution device 10, in an embodiment, provides for the vertically downward loading of the contents of vial 18 into the second piercing member 24 of cylindrical collar 16, whereby each of vial 18, the first piercing member 12 and the second piercing member 24 are oriented vertically. For example, when reconstitution device 10 is placed in an operating position, each of the first piercing member 12 and the second piercing member 24 is oriented vertically. In an embodiment, cylindrical collar 16 is oriented vertically such that, responsive to concentric engagement with the vial 18, the vial 18 is held vertically. By ensuring that the vial 18 is engaged with the reconstitution device 10 vertically (e.g., in a stopper-side down configuration), reconstituted material is readily encouraged to exit the vial 18 via gravity. Furthermore, a vertical orientation ensures that liquid reconstituted material may readily exit the vial 18, whereas foamy reconstituted material may float above the stopper-side of the vial, and thus above the second piercing member 24.

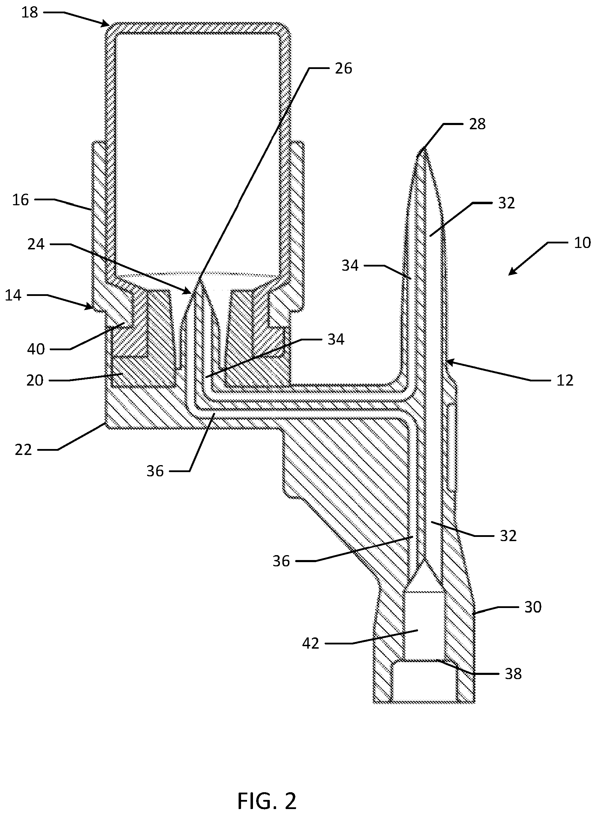

Fig. 2 is a sectioned view of the reconstitution device 10 of Fig. 1, further illustrating the engagement of device 10 according to an example embodiment of the present disclosure.

As illustrated, vial 18 is engaged in collar 16, such that the first end 26 of the second piercing member 24 pierces stopper 20 of vial 18. In one embodiment, the user pushes vial 18 down onto cap 22 to secure engagement between the vial 18 and the collar 16. The inner surface of collar 16 may include a plurality of ridges 40 that are configured to engage, e.g., via a springlike deformation, to press-fit or snap-fit over the stopper-side of vial 18, such that the stopper-side of vial 18 is retained inside the plurality of ridges 40 of the collar 16, as illustrated by Fig. 2. The the first end 26 of the second piercing member 24 pierces through the stopper 20 (e.g., a rubber stopper) of vial 18 and extends into vial 18. In a related embodiment, engagement between the collar 16 and the vial 18 may further include rotation or twisting of the vial 18. For example, the collar 16 may include inner threads, and the vial 18 may include outer threads, such that a threaded engagement between the vial 18 and the collar 16 is implemented for secure engagement between the components.

Once engaged, the first piercing member 12 is in fluid communication with the vial 18 via a second fluid pathway 34 that extends from the first end 28 of the first piercing member 12 to the first end 26 of the second piercing member 24. In this manner, vial 18 may be placed in fluid communication with an IV bag connected to first end 28 of the first piercing member 12. In an embodiment, the vial 18 is initially sealed and under a vacuum, prior to engagement with collar 16.

It should be appreciated that the piercing described above, with respect to each of the first end 28 of the first piercing member 12 and the first end 26 of the second piercing member 24 ideally happens nearly simultaneously. For example, once the first end 28 of first piercing member 12 pierces the administration port (e.g., an IV bag port), the first end 28 of the first piercing member 12 is in fluid communication with the administration port; thus, liquid could start flowing from the administration port into the first end 26 of the second piercing member 24 via the second fluid pathway 34. To ensure that liquid does not inadvertently spill out of the first end 26 of the second piercing member 24 (e.g., via second fluid pathway 34), the first end 26 of the second piercing member 24 should pierce stopper 20 of vial 18 almost immediately after the first end 28 of the first piercing member 12 pierces the administration port (e.g., nearly simultaneously). In this way, inadvertent spilling is prevented.

In an alternate embodiment, the reconstitution device 10 includes a valve. For example, the first end 28 of the first piercing member 12 includes the valve, which may be configured to completely seal off each of the first fluid pathway 32 and the second fluid pathway 34. When the valve is closed, and it seals off each of the first fluid pathway 32 and the second fluid pathway 34, piercing does not necessarily need to be nearly simultaneous (as previously noted). For example, once the first end 28 of first piercing member 12 pierces the administration port (e.g., an IV bag port), the first end 28 of the first piercing member 12 is in fluid communication with the administration port; however, the valve would prevent liquid from flowing from the administration port into the first end 26 of the second piercing member 24 via the second fluid pathway 34. The valve would ensure that liquid does not inadvertently spill out of the first end 26 of the second piercing member 24 (e.g., via second fluid pathway 34). Whenever the first end 26 of the second piercing member 24 pierces stopper 20 of vial 18, the valve would, likewise, ensure that the vacuum in vial 18 is retained. Consequently, once the valve is opened, and it unseals each of the first fluid pathway 32 and the second fluid pathway 34, fluid may flow into each of the first fluid pathway 32 and the second fluid pathway 34. In this way, inadvertent spilling is prevented; likewise, inadvertent loss of vacuum in vial 18 is prevented. It should be appreciated that the valve may be positioned at other locations on reconstitution device 10 and/or there could be multiple valves (e.g., one valve for each of first fluid pathway 32, second fluid pathway 34, and third fluid pathway 36).

Continuing on, in an embodiment, fluid, such as fluid in an IV bag, may flow directly from the first end 28 of first piercing member 12 to the second end 30 of first piercing member 12 via the first fluid pathway 32. Fluid may flow via the first fluid pathway 32 into the mixing chamber 42. Initially, fluid is prevented from flowing into an IV line by membrane 38. Once membrane 38 is removed or eliminated (e.g., pierced), mixing chamber 42 communicates with the IV line and fluid may flow via the first fluid pathway 32 to an IV line in communication with the second end 30 of the first piercing member 12. Fluid, such as fluid in an IV bag, may also gravity flow from the first end 28 of the first piercing member 12 to the vial 18 via the second fluid pathway 34. Fluid, such as a reconstituted solution in the vial 18, may flow from the vial 18 to the second end 30 of the first piercing member 12 via a third fluid pathway 36. Thus, in an example, fluid, such as saline or glucose from an IV bag, may travel into the first end 28 of the first piercing member 12 and subsequently into the vial 18 via second fluid pathway 34. The saline or glucose mixes with a dry, e.g., granulated, drug in vial 18 to form a reconstituted solution.

More specifically, and as described in greater detail below, fluid may initially flow into vial 18 from each of second fluid pathway 34 and third fluid pathway 36 as pressurized fluid streams (e.g., jet streams). Powdered drugs typically remain stuck to the base of the vial 18 which, when engaged with the reconstitution device 10 vertically in stopper-side down configuration, means that the powdered drugs are typically above the first end 26 of the second piercing member 24. The pressurized fluid streams may flow upward into vial 18, thus contacting the powdered drug to remove it from the base (e.g., top-side) of vial 18, to reconstitute it with the pressurized fluid, and to subsequently deliver it from vial 18 (e.g., via third fluid pathway 36) to a patient. Because vial 18 is oriented in a stopper-side down configuration, reconstituted drug may readily flow into the first end 26 of the second piercing member 24 (e.g., into the third fluid pathway 36) via gravity. For example, the reconstituted solution may flow from vial 18, through the third fluid pathway 36, out the second end 30 of the first piercing member 12 into a delivery site, such as an IV line.

It should be appreciated that the structural characteristics of each of the first fluid pathway 32, the second fluid pathway 34, and the third fluid pathway 36, may be dimensioned for performance to optimize, among other characteristics, hydraulic resistance. The first fluid pathway 32 may be configured as a parallel and independent bypass path straight to the infusion port. It is to be expected that design of the fluid pathways, including first fluid pathway 32, second fluid pathway 34, and third fluid pathway 36, can be configured for a particular hydraulic resistance of the fluid system, thus optimizing the infusion profile within vial 18. The dimensions and/or shape of the various fluid pathways may be adjusted (i.e., increase or decrease the diameter and/or increase or decrease the length and/or modify the cross-sectional shape of fluid pathways) to control and monitor the duration and/or the concentration of the infusion, according to the medical need related to the intended therapy application. For example, increasing the diameter of the first fluid pathway 32 would lead to a slower administration of the drug from the vial 18, as a lower proportion of fluid would be delivered to the vial 18 (e.g., via second fluid pathway 34) when compared to the first fluid pathway 32. It should be appreciated that the system disclosed herein may implement different fluid pathways for different fluids, different reconstituted drugs, and the like, to optimize delivery of any particular substance. Further, any one, or more, or all of fluid pathways 32, 34, 36 may be equipped with a manually operated flow restrictor to tailor flow rate to a particular treatment.

Providing multiple fluid pathways and thereby splitting or dividing flow from the first end 28 of first piercing member 12 to the second end 30 of first piercing member 12 may be beneficial, for example, to regulate the dosing of reconstituted solution (e.g., vitamins or pharmaceutical agents). In other words, there may be circumstances in which a bolus delivered to a patient may be too concentrated. Providing multiple fluid pathways at first end 28, some of which do not interact with vial 18 directly (e.g., first fluid pathway 32), results in a more moderate bolus that is diluted by reconstituted-free fluid (e.g., via first fluid pathway 32), thus extending or prolonging the administration of a composition.

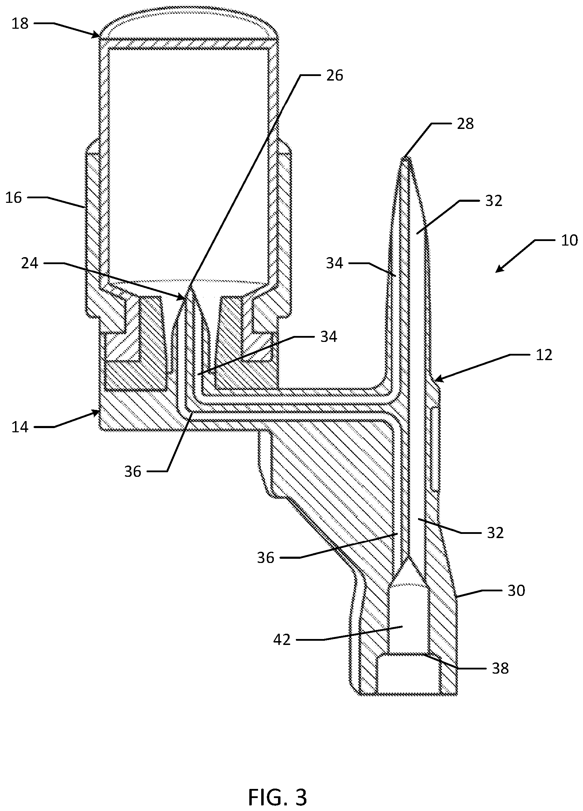

Fig. 3 is an elevation-sectioned view of the reconstitution device of Fig. 1, further illustrating device engagement, according to an example embodiment of the present disclosure. Collar 16 again may be cylindrical in nature to concentrically receive vial 18. An example method of using the reconstitution device 10 includes engaging the first end 28 of the first piercing member 12 with a fluid container, such as an IV bag. In an example, if the IV bag is a multi-chamber bag, the IV bag may require additional activation of the multi-chamber components, such as via bag-rolling or peel seal opening. In this way, a fluid may flow along the first fluid pathway 32 from the fluid container to the mixing chamber 42.

The method includes engaging the first end 26 of the second piercing member 24 with drug vial 18. In this way, the fluid may flow along the second fluid pathway 34 from the fluid container to the drug vial 18. Likewise, the fluid may flow along the first fluid pathway 32 to the mixing chamber 42, and then from the mixing chamber 42 along the third fluid pathway 36 to vial 18. During this initial mixing, fluid may be prevented from exiting the device 10 (e.g., into the IV line) by membrane 38. The method further includes mixing the fluid with an agent located within the drug vial 18, such as a dried drug or nutritional solution, to form a mixed or reconstituted drug or nutrition solution.

Lastly, the method includes engaging the second end 30 of the first piercing member 12 with an IV line. This may include, for example, removing the membrane 38. In this way, IV fluid may flow along the first fluid pathway 32 from the fluid container to the IV line. Likewise, the mixed or reconstituted drug or nutrition solution may flow along the third fluid pathway 36 from the drug vial 18 to the IV line.

In an embodiment, initially, the first end 28 of the first piercing member 12 pierces the fluid container. In this embodiment, the vial 18 is initially sealed and under a vacuum (e.g., a low pressure such as 100 mbar, which is less than ambient air pressure). When vial 18 is sealed under a vacuum, and then engaged by the first end 26 of the second piercing member 24, fluid from the fluid container will initially be rapidly drawn into the vial 18 by the negative pressure imbalance. This initial draw of fluid may generate a pressurized fluid stream from the first end 26 of the second piercing member 24 (e.g., a jet stream) into the vial 18. In certain embodiments, a pressurized fluid stream is advantageous for mixing and reconstituting a dried drug in the vial 18. The membrane 38 on the second end 30 of the first piercing member 12 further ensures that the reconstitution device 10 is airtight, such that pressurized fluid stream is not inadvertently dispensed out the second end 30 of the first piercing member 12. Membrane 38 may also encourage the initial filling of vial 18.

In an embodiment, the pressurized fluid stream (e.g., the jet stream) within the vial 18 is generated from each of the second fluid pathway 34 (as noted above) and the third fluid pathway 36. For example, the first piercing member 12 initially engages the fluid container. Consequently, when the first end 26 of the second piercing member 24 engages with the vial 18, a pressurized fluid stream may be pulled from each of the second fluid pathway 34 (e.g., from the fluid container to the vial 18) and the third fluid pathway 36 (e.g., from the fluid container to the mixing chamber 42 of the first piercing member 12, and then from the mixing chamber 42 of the first piercing member 12 to the vial 18) due to the lower pressure in the vial 18. In this way, reconstitution may occur via two pressurized fluid streams 34 and 36, dissolving, filling, and mixing the vial 18 contents within mere seconds. The magnitude of the vacuum in the vial 18 may be adjusted, to obtain an optimal jet effect. The vacuum also aids in reducing or eliminating problems in removing air if vial 18 instead contained under atmospheric pressure.

In an embodiment, an alternative auto-priming process may be performed for reconstitution device 10. For example, connection of the IV line to the second end 30 of the first piercing member 12 may occur prior to engaging the first end 26 of the second piercing member 24 with the drug vial 18. This results in an auto-priming effect, in which fluid is recalled from the IV line, which may have initially been derived from the IV bag, into the vial 18 via the vacuum to consequently fill both the vial 18 and a drip chamber associated with the IV line. In particular embodiments, auto-priming may require removal of membrane 38 prior to vial 18 engagement. The auto-priming process may advantageously eliminate any required squeezing of the IV line drip chamber to fill it, which is currently a typical step performed by medical professionals prior to IV administration.

In an alternate embodiment, the reconstitution device 10 may be implemented with an IV bag that is initially empty. For example, the reconstitution device 10 with vial 18 is connected to an empty IV bag (e.g., via administration port). The empty IV bag may then be subsequently filled with any desired solution, such as saline or glucose, through a dedicated port. This may be advantageous for applications where providing the solution ahead of time is costly or difficult. In this alternate embodiment, the solution for the IV bag may be prepared and the IV bag may be filled during administration. As solution is added to the empty IV bag (that is already connected to reconstitution device 10), reconstitution will occur as described above.

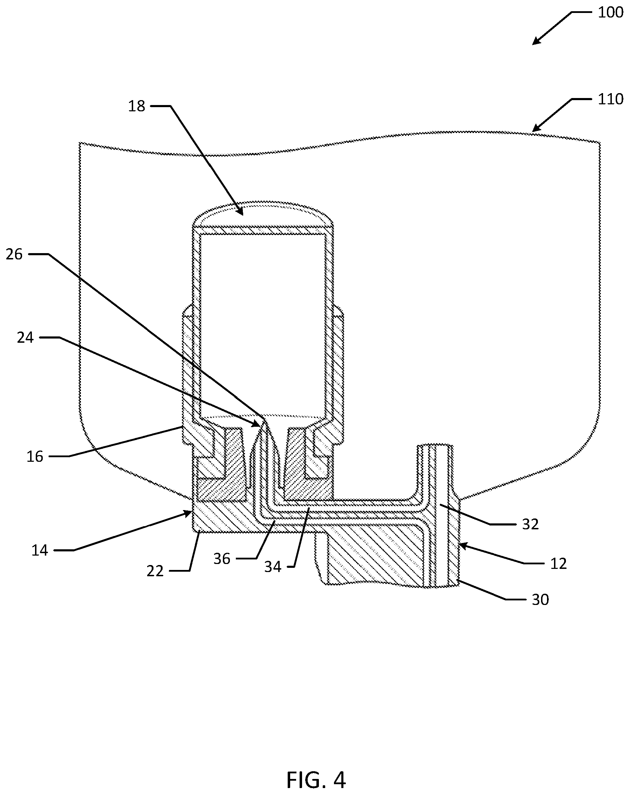

Fig. 4 shows another elevation-sectioned view of one alternative embodiment of a reconstitution device 100 of the present disclosure. Reconstitution device 100 as illustrated in Fig. 4 integrates fluid container **110,** which may be a rigid fluid container or a flexible fluid container, with the spike structure. In one embodiment, fluid container **110** is an IV bag. Fluid container 110 may, for example, be provided pre-loaded with a saline or glucose solution. The rigid portions of reconstitution device 100 may be made of any of the materials listed above for reconstitution device 10. Fluid container 110, if flexible, may be made of any suitable material known to those of skill in the art.

Reconstitution device 100 includes the first piercing member 12 and a receptacle 14. The receptacle 14 includes a cylindrical (or other shaped) collar 16. Cylindrical collar 16 is configured to engage a vial 18 (e.g., via concentric engagement). In the illustrated embodiment, the stopper-side of the vial 18 may be received within cylindrical collar 16, such that the cylindrical collar 16 is disposed around the outside of the stopper-side of vial 18. Vial 18 again may contain an active agent such as a pharmaceutical agent or a nutritional supplement. The active agent may be a dried powder, such as a powder obtained by lyophilization. It should be appreciated that, in alternative embodiments, the drug or active agent may be liquid. In other words, reconstitution device 10, 100 may be applicable for delivering both solids and liquids from vial 18 to the patient.

Receptacle 14 further includes a cap 22. Cap 22 may be formed integrally with or be attached to collar 16. Cap 22 extends from the collar 16 and holds a second piercing member 24. A first end 26 of second piercing member 24 is disposed within the collar 16. In an embodiment, at least one of the first end 28 of the first piercing member 12, the second end 30 of the first piercing member 12, and the first end 26 of the second piercing member 24 may include or form a syringe needle or a plastic spike.

The first end 28 (not shown) of first piercing member 12 is configured to engage the fluid container 110. In an embodiment, the first end 28 of the first piercing member 12 is formed integrally with the fluid container 110. The second end 30 of the first piercing member 12 is configured to engage an IV line. The first piercing member 12 includes the first fluid pathway 32 extending from the first end 28 of first piercing member 12 to the second end 30 of first piercing member 12, such that fluid container 110 is in fluid communication with the IV line. The second end 30 of the first piercing member 12 further includes the membrane (i.e., a removable membrane or a pierceable membrane).

The vial 18 engages collar 16, such that the first end 26 of second piercing member 24 extends into vial 18. Here, the first piercing member 12 is in fluid communication with vial 18 via the second fluid pathway 34 extending from the first end 28 of the first piercing member 12 to the first end 26 of the second piercing member 24. Vial 18 may accordingly be placed in fluid communication with the fluid container 110. In an embodiment, fluid container 110 holds the fluid, such as saline or glucose, which travels from fluid container 110 to the vial 18. Vial 18 may again be sealed initially and placed under a vacuum, prior to engagement with collar 16. In an embodiment, the vial 18 contains one of a pharmaceutical agent or a nutritional supplement.

Second piercing member 24 is in fluid communication with the IV line via third fluid pathway 36 extending from the first end 26 of second piercing member 24 to the second end 30 of first piercing member 12. Fluid from fluid container **110** may therefore travel into the first end 28 of the first piercing member 12 and subsequently into the vial 18. Membrane 38 (not shown) may be used to help backfill vial 18 with IV fluid from container 110. The fluid mixes with the dry, e.g., granulated, drug in vial 18 to form a reconstituted solution. Furthermore, for example, membrane 38 may define a common mixing chamber, whereby fluid from the first fluid pathway 32 (e.g., from fluid container 110) mixes with fluid from the third fluid pathway 36 (e.g., a reconstituted drug) prior to administration to a delivery location, such as the IV line. Once the drug has been reconstituted/mixed and is ready for delivery, membrane 38 may be removed or eliminated (e.g., pierced), so that the mixing chamber 42 communicates with the IV line. Once membrane 38 is removed or eliminated, the reconstituted solution may then flow from vial 18, through the third fluid pathway 36, out the second end 30 of the first piercing member 12, into the IV line.

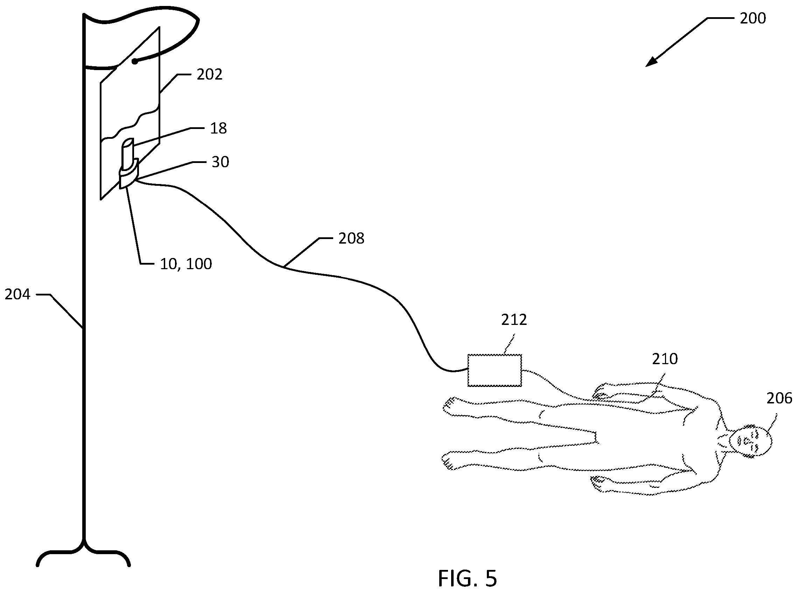

Fig. 5 illustrates one embodiment of a system 200 employing either reconstitution device 10 or reconstitution device 100, configured to engage with drug vial 18 as described above. System 200 includes an IV bag 202 hanging from a stand 204. The IV bag 202 may include a fluid, such as saline, glucose, or other similar fluid, for delivery to a patient 206. As illustrated, IV bag 202 is positioned on the stand 204 to ensure that the IV bag 202 is located vertically above the patient 206. This ensures that fluid in the IV bag 202 gravity flows to patient 206.

System 200 includes reconstitution device 10 (or 100). Reconstitution device 10 is connected to IV bag 202 as previously described (e.g., via first end 28 of first piercing member 12) and with vial 18 as previously described (e.g., via first end 24 of second piercing member 26). With device 100, IV bag 202 is formed integrally with the reconstitution device 100. System 200 also includes second end 30 of first piercing member. A delivery tube 208, such as an IV tube, engages with second end 30 of the first piercing member via any of the teachings listed above. Delivery tube 208 is also connected to patient 206 at an IV location 210. For example, delivery tube 208 extends to an intra-venous needle inserted into the patient 206 at the IV location 210.

Delivery tube 208 is configured to convey the fluid in the IV bag 202 from the reconstitution device 10 to the patient 206 at IV location 210. While fluid conveyance may be facilitated by gravity, as described above with respect to the IV bag 202 positioning on the stand 204, additional or alternative modes of fluid conveyance may be implemented. For example, system 200 may further include an infusion pump 212, which may for example be a Sigma^{™} Large Volume Pump ("LVP") provided by the assignee of the present disclosure. Infusion pump 212 may control fluid conveyance along delivery tube 208 (e.g., from the reconstitution device 10 to the patient 206 at the IV location 210). In such a case, bag 202 may be located vertically below patient 206.

With reference to Figs. 1 and 5, the fluid in the IV bag 202 may travel into the first end 28 of the first piercing member 12 and subsequently travel into the vial 18, via the second fluid pathway 34. The fluid mixes with the dried drug in the vial 18 to form a reconstituted solution. The reconstituted solution may then flow from the vial 18 to the second end 30 of the first piercing member 12, via the third fluid pathway 36. Simultaneously, fluid in the IV bag 202 may flow directly from the IV bag 202 to the second end 30 of the first piercing member 12 via the first fluid pathway 32. Consequently, IV fluid from IV bag 202 and reconstituted solution may both be delivered, from the second end 30 of the first piercing member 12, into delivery tube 208. IV fluid from the IV bag 202 and the reconstituted drug solution may then flow together to IV location 210 and be thereafter administered to the patient 206. In various embodiments, flow to the IV location 210 is conveyed via gravity and/or via infusion pump 212.

The fluid conveyance process described above may, in certain embodiments, include additional steps. For example, the reconstitution device 10 or 100 may engage the drug vial 18 at the cylindrical collar 16; however, prior to this engagement, the delivery tube 208 may be clamped (e.g., via a typical medical line clamp). The medical professional may do this to initially ensure that any inline air (e.g., in delivery tube 208 or in reconstitution device 10) is removed from the system 200. More particularly, once the reconstitution device 10 or 100 is affixed to both IV bag 202 and delivery tube 208, any inline air flows upwards into IV bag 202. Upward air flow may be facilitated by the IV bag 202 being positioned on the stand 204 so that it is vertically above the reconstitution device 10 or 100 and the patient 206. The medical professional may further ensure that no inline air remains via additional techniques (e.g., tapping the reconstitution device 10 or 100 and/or tube 208). Once all inline air has been purged to bag 202, the delivery tube 208 is unclamped and the fluid conveyance process described may begin.

A major concern for parenteral nutrition is that solutions administered peripherally to the patient (e.g., through peripheral veins) must remain under a certain limit of osmolarity. If the osmolarity is too high, the patient may experience pain or, in some cases, other adverse medical issues such as venial thrombosis. Therefore, the osmolarity of solutions administrated should be controlled and within safety limits. For example, a range of 850 mOsm/L to 900 mOsm/L is typical for an osmolarity limit.

An osmolarity study has been performed to quantify and monitor infusion administration of a nutritional compound to be administered with a Baxter PeriOlimel^{™} N4E multi-chamber bag (1000 mL volume) and the reconstitution device 10 disclosed herein. The nutrition compound, in a lyophilized or sterile powder, may contain the following vitamins:

| | |
|---|---|
| Retinol palmitate corresponding to Retinol (Vitamin A) | 3500 IU |
| Cholecalciferol (Vitamin D₃ ) | 200 IU |
| DL α-tocopherol | 10.2 mg |
| corresponding to α-tocopherol (Vitamin E) | 11.2 IU |
| Ascorbic Acid (Vitamin C) | 125 mg |
| Nicotinamide (Vitamin B₃ ) | 46 mg |
| Dexpanthenol | 16.15 mg |
| corresponding to pantothenic acid (Vitamin B₅) | 17.25 mg |
| Pyridoxine hydrochloride | 5.5 mg |
| corresponding to pyridoxine (Vitamin B₆) | 4.53 mg |
| Riboflavin sodium phosphate | 5.67 mg |
| corresponding to riboflavin (Vitamin B₂) | 4.14 mg |
| Cocarboxylase tetrahydrate | 5.8 mg |
| corresponding to thiamine (Vitamin B₁) | 3.51 mg |
| Folic Acid | 414 mcg |
| D-Biotin | 60 mcg |
| Cyanocobalamin (Vitamin B₁₂) | 5.5 mcg |

| ***Other Ingredients*** | |
|---|---|
| Glycine | 250 mg |
| Glycocholic acid | 140 mg |
| Soybean lecithin | 112.5 mg |

The reconstitution device 10 implemented in this study employed each of the first fluid pathway 32, the second fluid pathway 34, and the third fluid pathway 36 as described above. The first fluid pathway 32 was approximately 2.0 mm in diameter and 51 mm in length. Each of the second fluid pathway 34 and the third fluid pathway 36 were approximately 1.1 mm in diameter and 71 mm in length. It should be appreciated that other dimensions (e.g., for the diameter and length of each of first fluid pathway 32, second fluid pathway 34, and third fluid pathway 36 are contemplated). Each of the first fluid pathway 32, the second fluid pathway 34, and the third fluid pathway 36 had circular cross-sectional areas.

The following protocol was performed:
- Take a 1 mL aliquot of PeriOlimel^{™} N4E as a reference
- Start a drainage of PeriOlimel^{™} N4E + Nutrition Compound, attached to reconstitution device 10
- Collect aliquots of 1 mL every 10 minutes
- Stop drainage after 16 aliquots
- Open the Nutrition Compound vial, measure the volume of solution inside and a sample aliquot
- Measure osmolarity of all 18 aliquots (16 + reference + vial) using an osmeter and convert them to osmolarity (mOsm/L).

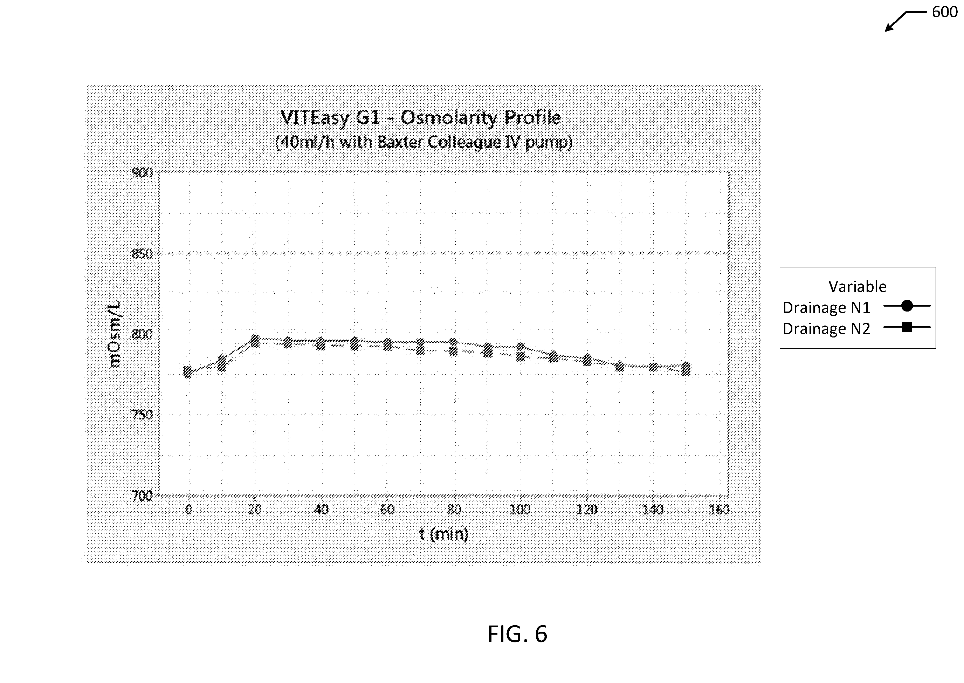

Fig. 6 is a plot of osmolarity profile as a function of time for the test above, with reconstitution device 10 of the present disclosure. The plot provides an illustration of an example performance of device 10, and is not intended to limit the foregoing disclosure in any respect. Plot 600 illustrates two separate tests, under the same experimental circumstances: Drainage N1 and Drainage N2.

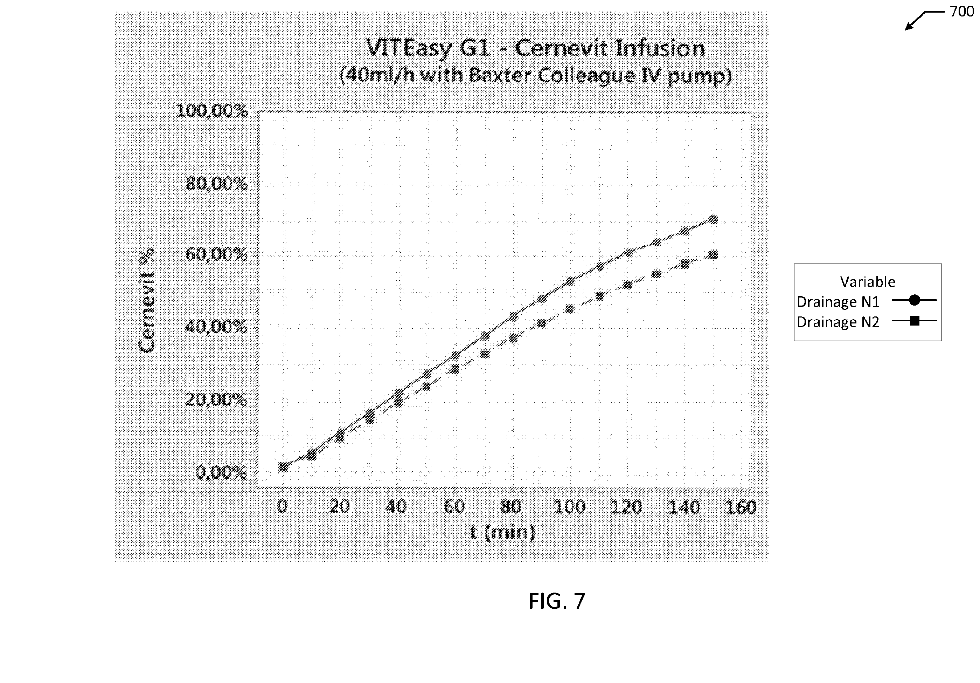

More particularly, with reference to Fig. 6, plot 600 shows that osmolarity resides at a maximum level of 800 mOsm/L at the beginning of administration, which is still below the typical guidelines for peripheral use. Osmolarity then slowly decreases as infusion time increases. Providing multiple fluid pathways, some of which do not interact with the vial 18 directly (e.g., first fluid pathway 32), enables reconstitution device 10 to achieve ideal osmolarity by providing a moderate bolus that is diluted by reconstituted-free fluid (e.g., via first fluid pathway 32), thus extending or prolonging the administration of a composition. With the osmolarity measurements of Fig. 6, the percentage of vitamins infused is determined for each time point and plotted in Fig. 7.

Fig. 7 is a plot of infusion as a function of time for the test above, with reconstitution device 10 of the present disclosure. The plot provides an illustration of an example performance of device 10, and is not intended to limit the foregoing disclosure in any respect. Plot 700 illustrates two separate tests, under the same experimental circumstances: Drainage N1 and Drainage N2.

More particularly, with reference to Fig. 7, plot 700 shows that infusion profiles of reconstitution device 10 are quite smooth. After 2.5 hours, approximately 60-70% of the vitamins had been infused, while about 30-40% remained. This smooth delivery profile confirms that delivery occurs moderately, avoiding a rapid bolus administration. Upon complete drainage of a full IV bag holding about 650 mL, approximately 98 % of the vitamins had been infused. In an embodiment, the drug vial used for infusion is formed of a UV light blocking material, to limit degradation of any reconstituted material, ensuring that reconstituted material is protected until it is infused (e.g., via an IV tube).

The above described reconstitution device, system, and method may be used, for example, during parenteral nutrition therapy. Here, reconstitution may be used to enhance the administration of a multivitamin product that would otherwise be added to a total parenteral nutrition ("TPN") bag through the medication port. Examples for known parenteral nutrition products, which could be used with the present device, system, and method, include Olimel, Oliclinomel, Clinomel, Clinimix, Numeta, ClinOleic, SmofKabiven, Kabiven, PeriKabiven, StructoKabiven, Aminomix, Nutriflex, Nutriflex Lipid, Pediaven, and the like. In various examples, the reconstitution device 10 or 100 may be permanently connected to a TPN bag, or may be added to the TPN bag prior to use. Likewise, either a multi-chamber bag or a singlechamber bag may be used with the present device, system, and method.

As used in this specification, including the claims, the term "and/or" is a conjunction that is either inclusive or exclusive. Accordingly, the term "and/or" either signifies the presence of two or more things in a group or signifies that one selection may be made from a group of alternatives.

## Claims

1. A reconstitution device (10) comprising:
a first piercing member (12) having a first end (28) and a second end (30), the first end of the first piercing member configured to engage an administration port of an IV fluid container and the second end of the first piercing member configured to be connected to an IV line;
a receptacle (14) including
a collar (16) configured to engage a vial (18), and
a cap (22) extending from the collar and holding a second piercing member (24) having a first end (26) disposed within the collar;
a first fluid pathway (32) extending from the first end (28) of the first piercing member (12) to the second end (30) of the first piercing member (12);
a second fluid pathway (34) extending from the first end (28) of the first piercing member (12) to the first end (26) of the second piercing member (24); and
a third fluid pathway (36) extending from the first end (26) of the second piercing member (24) to the second end (30) of the first piercing member (12);
wherein when the collar engages the vial, the first end (26) of the second piercing member (24) extends into the vial (18) and the first piercing member (12) is in fluid communication with the vial via the second fluid pathway (34),
wherein when the second end (30) of the first piercing member (12) is connected to the IV line, the first piercing member is in fluid communication with the IV line via the first fluid pathway and the second piercing member is in fluid communication with the IV line via the third fluid pathway, and
wherein the second end (30) of the first piercing member (12) further includes a removable membrane or a pierceable membrane (38).

2. The reconstitution device of claim 1, wherein the administration port is an intravenous ("IV") bag port, and wherein the first end (28) of the first piercing member (12) is configured to pierce the IV bag port to place the first piercing member in fluid communication with the IV bag port.

3. The reconstitution device of claim 1, further comprising an IV line.

4. The reconstitution device of claim 1, wherein, when placed in an operating position, each of the first piercing member (12) and the second piercing member (24) are oriented vertically.

5. The reconstitution device of claim 1, wherein the first end (28) of the first piercing member (12) is formed integrally with the administration port.

6. The reconstitution device of claim 1, wherein at least one of the first end (28) of the first piercing member (12), the second end (30) of the first piercing member (12), and the first end (26) of the second piercing member (24) includes or defines a syringe needle or a plastic spike.

7. The reconstitution device of claim 1, wherein the vial (18) is initially sealed and under a vacuum.

8. A reconstitution system (200) comprising:
a drug vial (18);
a fluid container (202); and
a reconstitution device (10) as claimed in claim 1.

9. The reconstitution system of claim 8, wherein the drug vial (18) contains one of a pharmaceutical agent or a nutritional supplement.

10. The reconstitution system of claim 8, wherein the drug vial (18) is initially sealed and under a vacuum.

11. The reconstitution system of claim 8, further comprising an infusion pump (212) in operable communication with the IV line.

12. The reconstitution system of claim 8, wherein the drug vial is formed of an ultraviolet ("UV") light blocking material.

13. A drug reconstitution method comprising:
providing a reconstitution device as claimed in claim 1;
enabling engagement of the first end (28) of the first piercing member (12) with a fluid container;
enabling engagement of the second end (30) of the first piercing member with an IV line, such that IV fluid may flow along the first fluid pathway (32) extending from the fluid container to the IV line;
enabling engagement of the first end (26) of the second piercing member (24) with a drug vial (18), such that IV fluid may flow along the second fluid pathway (34) extending from the fluid container to the drug vial;
enabling mixing of the IV fluid with an agent located within the drug vial (18) to form a mixed drug, such that the mixed drug may flow along the third fluid pathway (36) extending from the drug vial (18) to the IV line.

14. The reconstitution device of claim 1, wherein when the first end (28) of the first piercing member (12) is engaged with an IV fluid container, the second end (30) of the first piercing member is engaged with an IV line, and the collar is engaged with the vial (18) so that the first end (26) of the second piercing member (24) extends into the vial, IV fluid may flow along the first fluid pathway (32) from the IV fluid container to the IV line and along the second fluid pathway (34) from the IV fluid container to the drug vial (18) to mix with an agent located within the drug vial to form a mixed drug, and the mixed drug may flow along the third fluid pathway (36) from the drug vial (18) to the IV line, such that the IV fluid and the mixed drug may simultaneously flow from the second end (30) of the first piercing member (12) to the IV line.

## Patentansprüche

1. Rekonstitutionsvorrichtung (10), umfassend:
ein erstes Durchstechglied (12) mit einem ersten Ende (28) und einem zweiten Ende (30), wobei das erste Ende des ersten Durchstechglieds so konfiguriert ist, dass es in einen Verabreichungsanschluss eines IV-Flüssigkeitsbehälters eingreift, und das zweite Ende des ersten Durchstechglieds so konfiguriert ist, dass es mit einer IV-Leitung verbunden werden kann;
ein Aufnahme (14) einschließlich
eines Kragen (16), der so konfiguriert ist, dass er in eine Ampulle (18) eingreift, und
einer Kappe (22), die sich vom Kragen erstreckt und ein zweites Durchstechglied (24) mit einem ersten Ende (26), das innerhalb des Kragens angeordnet ist, hält;
eines ersten Flüssigkeitswegs (32), der sich vom ersten Ende (28) des ersten Durchstechglieds (12) zum zweiten Ende (30) des ersten Durchstechglieds (12) erstreckt;
eines zweiten Flüssigkeitswegs (34), der sich vom ersten Ende (28) des ersten Durchstechglieds (12) zum ersten Ende (26) des zweiten Durchstechglieds (24) erstreckt; und
eines dritten Flüssigkeitswegs (36), der sich vom ersten Ende (26) des zweiten Durchstechglieds (24) zum zweiten Ende (30) des ersten Durchstechglieds (12) erstreckt;
wobei, wenn der Kragen in die Ampulle eingreift, sich das erste Ende (26) des zweiten Durchstechglieds (24) in die Ampulle (18) erstreckt und das erste Durchstechglied (12) über den zweiten Flüssigkeitsweg (34) in Flüssigkeitsverbindung mit der Ampulle steht,
wobei, wenn das zweite Ende (30) des ersten Durchstechglieds (12) mit der IV-Leitung verbunden ist, das erste Durchstechglied über den ersten Flüssigkeitsweg in Flüssigkeitsverbindung mit der IV-Leitung steht und das zweite Durchstechglied über den dritten Flüssigkeitsweg in Flüssigkeitsverbindung mit der IV-Leitung steht, und
wobei das zweite Ende (30) des ersten Durchstechglieds (12) ferner eine entfernbare Membran oder eine durchstechbare Membran (38) einschließt.

2. Rekonstitutionsvorrichtung nach Anspruch 1, wobei der Verabreichungsanschluss ein intravenöser ("IV"). Beutelanschluss ist und wobei das erste Ende (28) des ersten Durchstechglieds (12) so konfiguriert ist, dass es den IV-Beutelanschluss durchsticht, um das erste Durchstechglied in Flüssigkeitsverbindung mit dem IV-Beutelanschluss zu bringen.

3. Rekonstitutionsvorrichtung nach Anspruch 1, ferner eine IV-Leitung umfassend.

4. Rekonstitutionsvorrichtung nach Anspruch 1, wobei in einer Betriebsposition sowohl das erste Durchstechglied (12) als auch das zweite Durchstechglied (24) vertikal ausgerichtet sind.

5. Rekonstitutionsvorrichtung nach Anspruch 1, wobei das erste Ende (28) des ersten Durchstechglieds (12) einstückig mit dem Verabreichungsanschluss gebildet ist.

6. Rekonstitutionsvorrichtung nach Anspruch 1, wobei mindestens eines der ersten Enden (28) des ersten Durchstechglieds (12), der zweiten Enden (30) des ersten Durchstechglieds (12) und der ersten Enden (26) des zweiten Durchstechglieds (24) eine Spritzennadel oder einen Kunststoffdorn einschließt oder definiert.

7. Rekonstitutionsvorrichtung nach Anspruch 1, wobei die Ampulle (18) zu Beginn versiegelt ist und unter Vakuum steht.

8. Rekonstitutionssystem (200), umfassend:
eine Arzneimittelampulle (18);
einen Flüssigkeitsbehälter (202); und
eine Rekonstitutionsvorrichtung (10) nach Anspruch 1.

9. Rekonstitutionssystem nach Anspruch 8, wobei die Arzneimittelampulle (18) entweder ein pharmazeutisches Mittel oder ein Nahrungsergänzungsmittel enthält.

10. Rekonstitutionssystem nach Anspruch 8, wobei die Arzneimittelampulle (18) zu Beginn versiegelt ist und unter Vakuum steht.

11. Rekonstitutionssystem nach Anspruch 8, ferner eine Infusionspumpe (212) umfassend, die in funktionsfähiger Verbindung mit der IV-Leitung steht.

12. Rekonstitutionssystem nach Anspruch 8, wobei die Arzneimittelampulle aus einem ultraviolettes ("UV") Licht blockierenden Material gebildet ist.

13. Rekonstitutionsverfahren von Arzneimitteln, umfassend:
Bereitstellen einer Rekonstitutionsvorrichtung nach Anspruch 1;
Ermöglichen des Eingriffs des ersten Endes (28) des ersten Durchstechglieds (12) mit einem Flüssigkeitsbehälter;
Ermöglichen des Eingriffs des zweiten Endes (30) des ersten Durchstechglieds mit einer IV-Leitung, so dass IV-Flüssigkeit entlang des ersten Flüssigkeitswegs (32) fließen kann, der sich vom Flüssigkeitsbehälter zur IV-Leitung erstreckt;
Ermöglichen des Eingriffs des ersten Endes (26) des zweiten Durchstechglieds (24) mit einer Arzneimittelampulle (18), so dass die IV-Flüssigkeit entlang des zweiten Flüssigkeitswegs (34) fließen kann, der sich vom Flüssigkeitsbehälter zur Arzneimittelampulle erstreckt;
Ermöglichen des Mischens der IV-Flüssigkeit mit einem in der Arzneimittelampulle (18) befindlichen Wirkstoff, um ein gemischtes Arzneimittel zu bilden, so dass das gemischte Arzneimittel entlang des dritten Flüssigkeitswegs (36) fließen kann, der sich von der Arzneimittelampulle (18) zur IV-Leitung erstreckt.

14. Rekonstitutionsvorrichtung nach Anspruch 1, wobei, wenn das erste Ende (28) des ersten Durchstechglieds (12) mit einem IV-Flüssigkeitsbehälter in Eingriff steht, das zweite Ende (30) des ersten Durchstechglieds mit einer IV-Leitung in Eingriff steht und der Kragen so mit der Ampulle (18) in Eingriff steht, dass sich das erste Ende (26) des zweiten Durchstechglieds (24) in die Ampulle erstreckt, die IV-Flüssigkeit entlang des ersten Flüssigkeitswegs (32) vom IV-Flüssigkeitsbehälter zur IV-Leitung und entlang des zweiten Flüssigkeitswegs (34) vom IV-Flüssigkeitsbehälter zur Arzneimittelampulle (18) fließen kann, um sich mit einem in der Arzneimittelampulle befindlichen Wirkstoff zu vermischen und ein gemischtes Arzneimittel zu bilden, und das gemischte Arzneimittel entlang des dritten Flüssigkeitswegs (36) von der Arzneimittelampulle (18) zur IV-Leitung fließen kann, damit die IV-Flüssigkeit und das gemischte Arzneimittel gleichzeitig vom zweiten Ende (30) des ersten Durchstechglieds (12) zur IV-Leitung fließen können.

## Revendications

1. Dispositif de reconstitution (10) comprenant :
un premier élément de perçage (12) ayant une première extrémité (28) et une seconde extrémité (30), la première extrémité du premier élément de perçage étant configurée pour entrer en prise dans un port d'administration d'un récipient de fluide IV et la seconde extrémité du premier élément de perçage étant configurée pour être connectée à une ligne IV ;
un réceptacle (14) comportant
un collier (16) configuré pour entrer en prise dans un flacon (18), et
un capuchon (22) se prolongeant du collier et contenant un second élément de perçage (24) ayant une première extrémité (26) disposée à l'intérieur du collier ;
un premier chemin de fluide (32) se prolongeant de la première extrémité (28) du premier élément de perçage (12) à la seconde extrémité (30) du premier élément de perçage (12) ;
un deuxième chemin de fluide (34) se prolongeant de la première extrémité (28) du premier élément de perçage (12) à la première extrémité (26) du second élément de perçage (24) ; et
un troisième chemin de fluide (36) se prolongeant de la première extrémité (26) du second élément de perçage (24) à la seconde extrémité (30) du premier élément de perçage (12) ;
dans lequel, lorsque le collier entre en prise dans le flacon, la première extrémité (26) du second élément de perçage (24) se prolonge dans le flacon (18) et le premier élément de perçage (12) est en communication fluidique avec le flacon par le biais du deuxième chemin de fluide (34),
dans lequel, lorsque la seconde extrémité (30) du premier élément de perçage (12) est connectée à la ligne IV, le premier élément de perçage est en communication fluidique avec la ligne IV par le biais du premier chemin de fluide et le second élément de perçage est en communication fluidique avec la ligne IV par le biais du troisième chemin de fluide, et
dans lequel la seconde extrémité (30) du premier élément de perçage (12) comporte également une membrane amovible ou une membrane perforable (38).

2. Dispositif de reconstitution selon la revendication 1, dans lequel le port d'administration est un port de poche intraveineuse (« IV »), et dans lequel la première extrémité (28) du premier élément de perçage (12) est configurée pour percer le port de la poche IV afin de placer le premier élément de perçage en communication fluidique avec le port de la poche IV.

3. Dispositif de reconstitution selon la revendication 1, comprenant également une ligne IV.

4. Dispositif de reconstitution selon la revendication 1, dans lequel, lorsqu'il est placé en position de fonctionnement, chacun du premier élément de perçage (12) et du second élément de perçage (24) sont orientés verticalement.

5. Dispositif de reconstitution selon la revendication 1, dans lequel la première extrémité (28) du premier élément de perçage (12) est formée intégralement avec le port d'administration.

6. Dispositif de reconstitution selon la revendication 1, dans lequel au moins l'une de la première extrémité (28) du premier élément de perçage (12), de la seconde extrémité (30) du premier élément de perçage (12) et de la première extrémité (26) du second élément de perçage (24) comporte ou définit une aiguille de seringue ou une pointe en plastique.

7. Dispositif de reconstitution selon la revendication 1, dans lequel le flacon (18) est initialement scellé et sous vide.

8. Système de reconstitution (200) comprenant :
un flacon de médicament (18) ;
un récipient à fluide (202) ; et
un dispositif de reconstitution (10) tel que revendiqué selon la revendication 1.

9. Système de reconstitution selon la revendication 8, dans lequel le flacon de médicament (18) contient l'un d'un agent pharmaceutique ou d'un complément nutritionnel.

10. Système de reconstitution selon la revendication 8, dans lequel le flacon de médicament (18) est initialement scellé et sous vide.

11. Système de reconstitution selon la revendication 8, comprenant également une pompe à perfusion (212) en communication fonctionnelle avec la ligne IV.

12. Système de reconstitution selon la revendication 8, dans lequel le flacon de médicament est formé d'un matériau bloquant la lumière ultraviolette (« UV »).

13. Procédé de reconstitution d'un médicament comprenant :
la fourniture d'un dispositif de reconstitution tel que revendiqué selon la revendication 1 ;
l'entrée en prise de la première extrémité (28) du premier élément de perçage (12) avec un récipient de fluide ;
l'entrée en prise de la seconde extrémité (30) du premier élément de perçage avec une ligne IV, de sorte que le fluide IV puisse circuler le long du premier chemin de fluide (32) se prolongeant du récipient de fluide à la ligne IV ;
l'entrée en prise de la première extrémité (26) du second élément de perçage (24) avec un flacon de médicament (18), de sorte que le fluide IV puisse s'écouler le long du deuxième chemin de fluide (34) se prolongeant du récipient de fluide au flacon de médicament ;
le mélange du fluide IV avec un agent situé dans le flacon de médicament (18) pour former un médicament mélangé, de sorte que le médicament mélangé puisse circuler le long du troisième chemin de fluide (36) se prolongeant du flacon de médicament (18) à la ligne IV.

14. Dispositif de reconstitution selon la revendication 1, dans lequel lorsque la première extrémité (28) du premier élément de perçage (12) est en prise avec un récipient de fluide IV, la seconde extrémité (30) du premier élément de perçage est en prise avec une ligne IV, et le collier est en prise avec le flacon (18) de sorte que la première extrémité (26) du second élément de perçage (24) se prolonge dans le flacon, le fluide IV peut s'écouler le long du premier chemin de fluide (32) du récipient de fluide IV vers la ligne IV et le long du deuxième chemin de fluide (34) du récipient de fluide IV vers le flacon de médicament (18) pour se mélanger avec un agent situé dans le flacon de médicament afin de former un médicament mélangé, et le médicament mélangé peut s'écouler le long du troisième chemin de fluide (36) du flacon de médicament (18) vers la ligne IV, de sorte que le fluide IV et le médicament mélangé peuvent s'écouler simultanément de la seconde extrémité (30) du premier élément de perçage (12) vers la ligne IV.
